# EUROPEAN PATENT APPLICATION

(11) **EP 2 695 875 A1**
(43) Date of publication of application: **12.02.2014**
(21) Application number: 12005784.9
(22) Date of filing: 09.08.2012
(51) Int. Cl.: C07C 51/265, C07B 33/00, C07B 41/08, C07B 41/14, C07C 407/00, C07C 409/10, C07C 63/26, C07C 63/06

(54) **Improved method for the oxidation of alkyl aromatic hydrocarbons**

(71) Applicant: Linde Aktiengesellschaft, 80331 München (DE)
(72) Inventor: Obermeyer, Heinz-Dieter, Dr., 85356 Freising (DE); Reinhardt, Hans-Jürgen, Dr., 87600 Kaufbeuren (DE); Schreiner, Bernhard, Dr., 82041 Oberhaching (DE); Wolf, Stefan, Dr., 85540 Haar (DE)
(74) Representative: Kasseckert, Rainer

(57) **Abstract**

The present invention concerns an improved method for oxidation of alkyl aromatic hydrocarbons in a tank reactor system wherein the oxygen content in the oxidation gas is 21.5 to 30 % by volume.

## Description

The present invention relates to an improved method for the oxidation of alkyl aromatic hydrocarbons wherein the alkyl aromatic hydrocarbons are reacted with an oxidation gas,.

The oxidation of alkyl aromatic hydrocarbons is an important reaction in industrial organic chemistry. These reactions are gas-liquid reactions wherein the oxidation of hydrocarbons with molecular oxygen plays a major role in this. It is used to produce both bulk products as well as fine and specialty chemicals. Table 1 below provides an overview of the most important reactions and their production capacities.

| **Oxidation of** | **Product** | **Production 2004 in Europe i n kt/a** | **Production 2004 globally i n kt/a** |
|---|---|---|---|
| **p-xylene** | **purified terephthalic acid (PTA)** | **3,180** | **30,640** (2003) |
| **p-xylene** | **dimethyl terephthalate (DMT)** | **1,865** | **4,928** (2003) |
| **cyclohexane** | **KA oil (cyclohexanone-cyclohexanol mixture)** | **2,380** | **5,734** |
| **cumene and subsequent cleavage of hydroperoxide** | **acetone + phenol** | **2,000 acetone 3,277 phenol** | **4,937acetone 8,093 phenol** |
| **cyclohexane** | **adipic acid** | **802** | **2,255** (2006) |
| **ethylene** | **acetaldehyde** | **212** | **1,600** |
| **acetaldehyde** | **acetic acid** | **100** | **737** (2006) |
| **oxo-aldehydes** | **oxo-carbonic acids (for example: n-butyric acid, isooctanoic acid)** | **3,422** | **9,587** (2005) |

These reactions, however, often suffer under low selectivity and high CO₂ emission.

Thus, it is object of the present invention to improve the known industrial processes for oxidizing alkyl aromatic hydrocarbons, in particular cumene, toluene and p-xylene, with regard to higher selectivity and lower emission of pollution gases.

This objection is solved by a process as claimed in claim 1. Preferred embodiments are the subject matter of the dependent claims.

The majority of homogenous oxidation reactions use air for oxidation.

The present inventors now have recognized that the known processes for oxidizing alkyl aromatic hydrocarbons are improved by lowering the reaction temperature concurrently with oxygen enrichment.

As used herein, the term "alkyl aromatic hydrocarbon" means any cyclic aromatic C₅-C₁₀-system having one, two or more C₁- C₆ alkyl group substituent (preferably methyl or ethyl). Preferred alkyl aromatic compounds are cumene, toluene or p-xylene, m-xylene and ethylbenzene.

In the present invention oxygen enrichment in the oxidation gas up to 30% by volume has been investigated. It has been found advantageous to use from 21.5% by volume up to 23% by volume, up to 24% by volume, up to 25% by volume, up to 26% by volume or up to 27% by volume oxygen in the oxidation gas. Preferably the oxygen content in the oxidation gas is 23-30% by volume, 24-30% by volume, 25-30% by volume, 26-30% by volume, more preferably 24% by volume, 25% by volume, 26% by volume or 27% by volume. The oxidation gas may be oxygen-enriched air or technical oxygen.

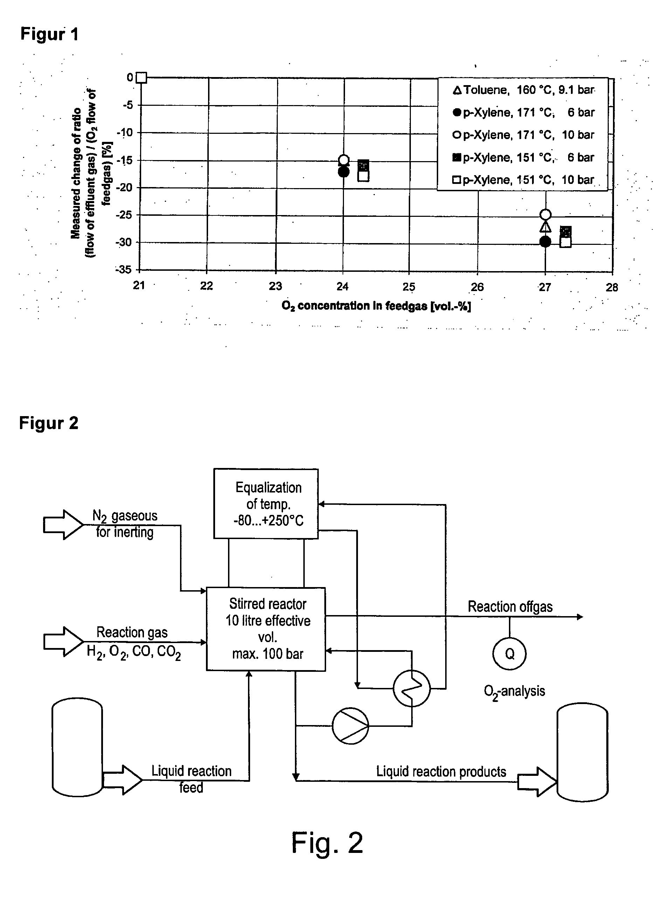

For example, when keeping the oxidation gas flow to the reactor on the same level as applied in air-only mode, Figure 1 shows how elevated oxygen content reduces the effluent gas flow in oxidation of cumene, toluene, and p-xylene. Evidently, oxygen enrichment to 24% by volume causes a reduction by about 15%; enrichment to 27% by volume reduces the effluent gas flow by approximately 30%.

Extensive experience with technical-scale units applying air oxidation steps (e.g.: Claus process, FCC process and gas/liquid oxidation of p-xylene or cyclohexane) shows that oxygen enrichment of up to 30% by volume or up to 27% by volume can usually be introduced at minimal cost and handled efficiently as well as safely.

The experiments were performed in a test unit as described for example in Fig. 2. The test unit can be operated in batch and semi-batch mode or continuously. The experiments are preferably performed in the semi-batch-mode; i.e. a given volume of approx. 10 litres of liquid feed was aerated by a steady flow of oxidation gas, a mixture of pure nitrogen and oxygen.

Operational data from an existing technical oxidation reactor operated in the "air-only mode" were used as the foundation for testing the program. After the model was successfully traced, preliminary calculations were performed probing oxygen-enriched air operation. The calculations were executed for, among other things, a bubble column with internal heat exchangers. For safety reasons, operators typically set a limiting concentration of oxygen in the effluent gas of < 5% by volume for air oxidation. This is sufficiently far away from the "real" limit of 9.8% by volume that was measured at 5 bar and 100 °C. Accordingly, the calculations were performed setting the basic condition that the oxygen content in the effluent gas may not exceed preferably 4.2% by volume.

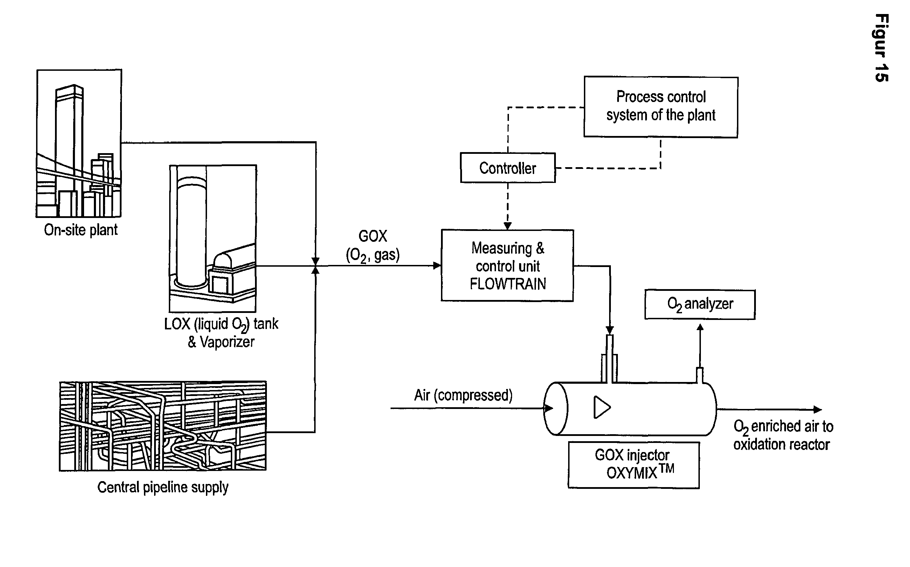

The technical issues related to introduction of oxygen enrichment are relatively minor up to an oxygen enrichment to a concentration of about 21.5-30% by volume. Figure 15 shows preferred options for supplying oxygen and is a preferred basic diagram for achieving oxygen enrichment. In addition to the supply of oxygen which can be delivered in liquid form, preferably through an on-site plant or a piping system, only very limited changes are necessary. The measurement and control unit ensures safe and reliable dosing of gaseous oxygen during the reactor's or system's various operational states. Accordingly, this unit has a block and bleed system that e.g. prevents oxygen from entering the piping leading to the reactor when the air is shut off. The oxygen dosing station also establishes the connection to the process control system.

The preferably used oxygen injector OXYMIX™ mentioned in Fig. 15 is designed to ensure that the gaseous oxygen is properly mixed with the air. The mixing should preferably occur over a short distance, the mixture should be as homogenous as possible, and the oxygen does not directly hit the wall of the air duct.

Proper distribution of the gas in the reactor is preferred in order to avoid inhomogenities in the reactor. To achieve this, a suitable gas distribution system and effective mixing of the gas and liquid phases by the rising bubbles or the stirrer, respectively, should take place. In some cases, it may be necessary to replace the gas distributor in order to operate the reactor safely. To ensure safety where the oxidation air enters the liquid reaction mass, the possibility of the reaction mass flowing back into the gas distributor should be prevented. This can be achieved by feeding nitrogen when the air stops and by properly arranging the outlet holes. Since oxygen enrichment increases the speed of the reaction, the amount of produced heat increases during these exothermic oxidations. Therefore, it can be necessary to increase the capacity of the heat exchanger in order to safely control the balance of heat.

As shown in Figure 15, gaseous oxygen (GOX) can be provided through a supply based on liquid oxygen (LOX), an on-site plant, or a connection to an existing piping network. The following Table 2 provides an overview of the options.

| **Oxygen requirement in Nm³/h** | **Purity [vol.-%]** | **How gaseous oxygen is provided** | **Demands on the supply** |
|---|---|---|---|
| 0 - approx. 1,000 | > 99.5 | LOX delivered by truck to tank/evaporator system | Consumption volumes vary greatly |
| approx. 300-5,000 | < 94 | O₂-PSA (VPSA), on-site supply | Load range typically 30% to 100% |
| approx. 1,000-100,000 | > 99.5 | Cryogenic air separator, on-site supply | Load range typically 50% to 100% |

In many cases, a PSA system provides an adequate oxygen quality, because very high oxygen purity is not necessary for oxygen enrichment. If oxygen is used only occasionally or mostly for experimental periods, a supply based on LOX is often advisable. The benefits hereby are the ability to install it quickly and at low cost besides very flexible utilization options; e.g. when facing highly fluctuating oxygen demand.

In summary, the following items that should be considered when analyzing technical safety during air oxidation, especially when introducing oxygen enrichment, include:
- When the oxygen-enriched air enters the reactor, its temperature should, where possible, be lower than the initialization temperature of the reaction in order to avoid the dangers of oxidation in the gas distributor area if penetrated by liquid.
- Avoidance of uncontrolled reactions, especially oxidations and thermal decomposition of peroxides, such as those that may appear during cumene oxidation.
- Prevention of the formation of a larger-volume combustible gas phase of inflammable composition. This is especially true for the reactor head space, where the limiting concentration of oxygen must not be exceeded. This is often at 9% to 11% by volume. To implement a safety margin for technical operation the max. allowed limit of oxygen content is usually set art ≤ 5% by volume.
- In terms of the bubbles, it is assumed that they may explode, because the resulting energy is low and is absorbed by the liquid phase. Thus, the reaction conditions in the reactor should be set in a way that the oxygen concentration in the bubbles reaching the pure gas phase - i.e. in the reactor's head-space - is below the selected limiting concentration of oxygen, such as 5% by volume.
- Potentially dangerous operating conditions appear when starting and stopping oxidation reactors. For this reason, special measures must be defined. This may include the use of nitrogen when starting and stopping the reactor.
- When introducing oxygen enrichment, the materials and seals in the relevant pipes and apparatus should be checked. Changes are not usually required when the concentration of oxygen in the oxidation air is 27% by volume or lower. Preferably, the auto-ignition temperatures and upper and lower flammability limits of the hydrocarbons should be taken into consideration. Since oxygen enrichment normally does not require any major changes to the reaction conditions compared to air oxidation, in most cases the resulting requirements are fulfilled already.

The following effects can be achieved through oxygen enrichment of the oxidation air:
- Reduction of the effluent gas volume; i.e. less hydrocarbon losses and mitigated generation of carbon dioxide emissions
- Elevated production capacity by allowing for elevated throughput of liquid.
- Improved selectivity in dependence of the given reaction system, e.g. higher reaction speed resulting from the increased oxygen partial pressure allows for the application of lowered oxidation temperature.

In economic terms process intensification by oxygen enrichment means:
- Higher productivity through elevated yields and throughput.
- Lower operating costs, especially in terms of energy costs for air compression and effluent gas treatment.
- Financial benefits when trading CO₂ certificates.
- Lower investment costs when new plants' design is based on oxygen enrichment.

The present invention is further described by the accompanying figures:
- Figure 1:: Reduction of effluent gas flow measured in a stirred tank reactor as oxygen concentration in the oxidation air (feed gas) increases for cumene, toluene, and p-xylene oxidation
- Figure 2:: Basic diagram of the applied stirred tank reactor system
The modular unit consists primarily of the following components:
- Dosing
   Gas dosing, storage container, high-pressure dosing pump (5 to 50 Uh)
- Stirred tank reactor with external circulation loop
- Temperature control unit (-80 to +250 °C)
- Operating panel with process control system
- Figure 3:: (a) Steps of Cumene-Phenol-Process
(b) Reaction kinetics
(c) Calculation of the conversion and selectivity
- Figure 4:: Influence of the oxygen content in the oxidation air on conversion of cumene at different reaction temperatures at 3 barg
- Figure 5:: Relative change of cumene conversion with increasing oxygen enrichment
- Figure 6:: Selectivity with respect to cumene hydroperoxide at different temperatures and oxygen contents in the oxidation air (3 barg)
- Figure 7:: Toluene oxidation to benzoic acid
- Figure 8:: Yield of benzoic acid and by-products during toluene oxidation (160°C, 9 bar, O₂ content of 27% by volume in the oxidation gas)
- Figure 9:: Accumulated oxygen consumption and oxygen concentration in effluent gas during toluene oxidation (160°C, 9 bar; O₂ content of 27% by volume in the oxidation gas)
- Figure 10:: Main reactions in the Witten process for production of DMT
- Figure 11:: Measured yields of MTT at various oxygen concentrations in the oxidation air (170°C, 6 bar)
- Figure 12:: Measured yields of p-TA at various oxygen concentrations in the oxidation air (170°C, 6 bar)
- Figure 13:: Progression of oxygen content in the effluent gas and oxygen consumption at various reaction times (170°C, 6 bar)
- Figure 14:: Selectivity to MTT at various oxygen concentrations in the oxidation air
- Figure 15:: Basic diagram for oxygen enrichment
- Figure 16:: Oxidation of p-xylene to terephthalic acid

In the following sections the invention is described in further detail as regards the experimental investigations with additional use of oxygen in air oxidation of cumene, toluene, and p-xylene. In addition, the technical measures necessary for introducing oxygen enrichment, provision of oxygen, and safety questions are explored.

### Cumene oxidation

The oxidation of cumene to cumene hydroperoxide is an intermediate step in the production of phenol and acetone. This involves autocatalytic air oxidation that is usually performed in stirred tank reactors or bubble columns. It is a technically very important method.

Since cumene hydroperoxide is an intermediate product, one has to look at one of the downstream products - e.g. phenol - to obtain market figures. For the future growth in demand of 2% per year is expected (ICIS Chemical Business 40: "Chemical Profile: Phenol", November 17-30, 2008).

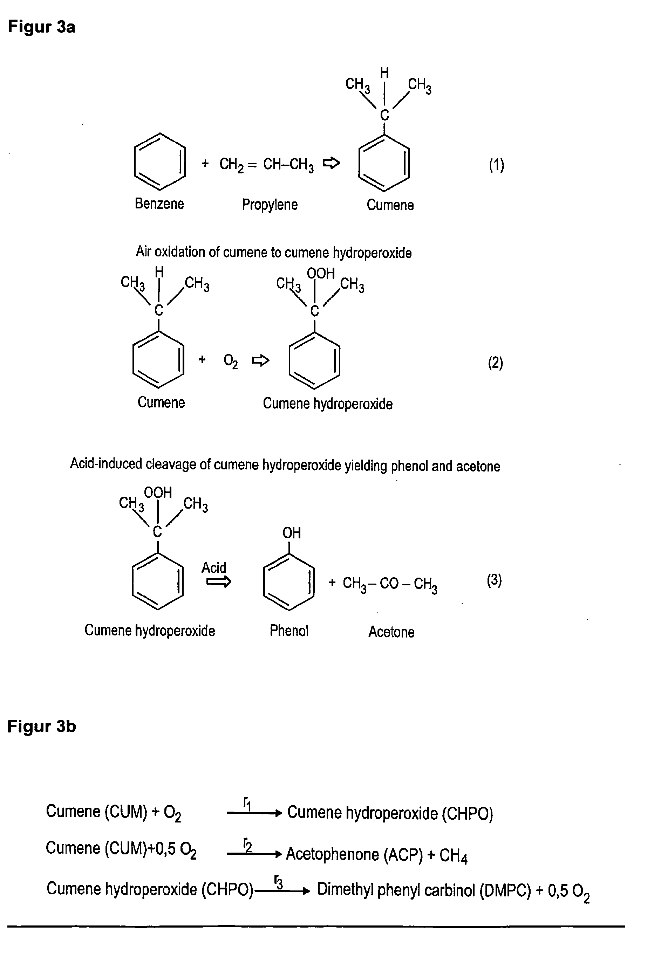

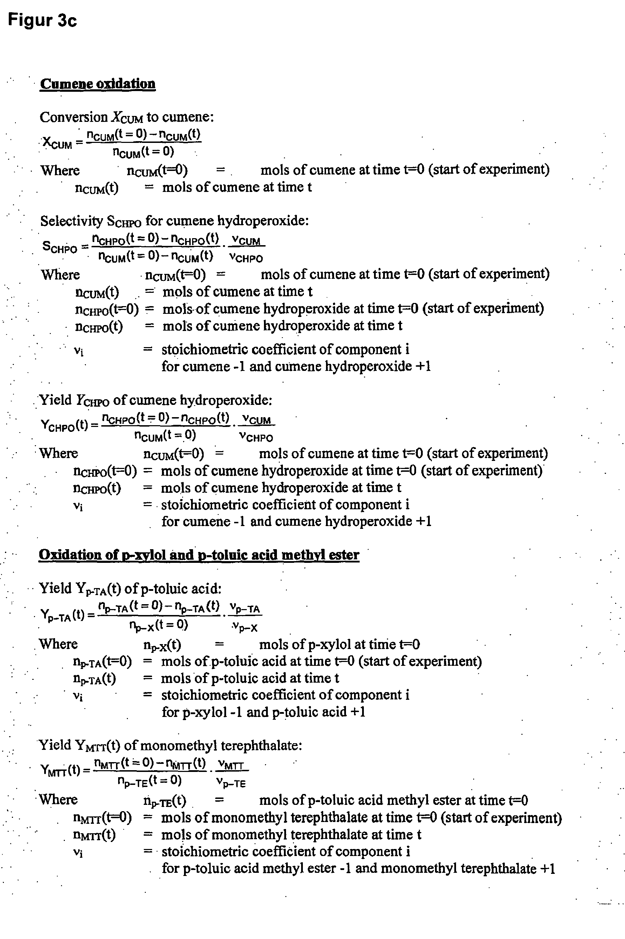

The Cumene-Phenol-Process (Ullmann's Encylopedia of Industrial Chemistry, Volume A 19, "Phenol", 1991, 5th ed. VCH-Verlag, pp. 299-312) which contains the oxidation of cumene as the initial step, was developed by Hock and Lang in 1944. The first plant with a capacity of 8,000 metric tons per year was constructed in Canada in 1953. Despite the unavoidable production of 0.62 metric tons of acetone per metric ton of phenol, the process remains by far the most important production process. It is based on three reaction steps as shown in Fig. 3a:
(1.) Alkylation of benzene with propylene to cumene
(2.) Air oxidation of cumene to cumene hydroperoxide
(3.) Acid-induced cleavage of cumene hydroperoxide yielding phenol and acetone

Investigating the cumene oxidation step (2) the experimental conditions were selected according to reaction conditions applied in the technical scale. The experiments were mainly performed in the stirred reactor system described in Figure 2 in semi-batch mode under the following reaction conditions:

| | |
|---|---|
| Temperature: | 105 - 125 °C |
| Pressure: | 3 - 5 bar |
| Oxygen content of the oxidation gas: | 21 to 27% by volume |
| Initial concentration of cumene hydro peroxide: | 3 to 5% by mass |

In addition, both a stirred tank reactor and a bubble column were used as oxidator basis for devising and programming of a common reactor model. It was assumed that heat produced by the reaction can be removed by heat exchangers in the reactor and/or in a closed external loop attached to it. In most technical installations, two to five stirred tank reactors or bubble columns are installed in series and can also be operated at different temperatures. This makes it possible to keep the production of by-products on a low level. Since oxidation proceeds slowly at low temperatures, resulting in several hours of residence time, the largest possible reactors, such as those with a reaction volume of more than 300 m³, are used to keep investment costs as low as possible.

Keeping the flow of oxidation gas to the stirred vessel on a constant level the oxygen content in this gas stream was raised step-wise from 21 to 27% by volume in order to investigate correlated effects on the oxidation. Cumene, cumene hydroperoxide, acetophenone, dimethyl phenyl carbinol, and dicumyl peroxide were detected analytically. Analysis of the effluent gas detected predominantly methane and oxygen content therein.

Experimental results clearly show that oxygen enrichment causes the following effects:
- Cumene conversion increases as the oxygen content in the oxidation air grows.
- Cumene hydroperoxide yield also increases as oxygen content increases.
- The formation of byproducts, in particular acetophenone, dimethyl phenyl carbinol, and dicumyl peroxide changes to an only minor degree as the oxygen content increases.

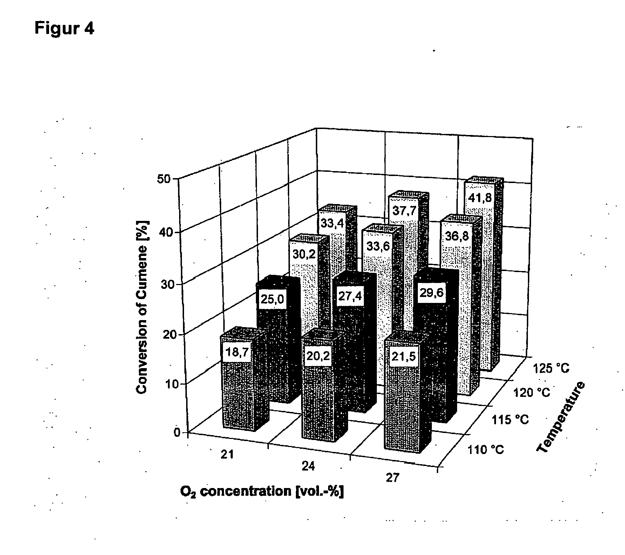

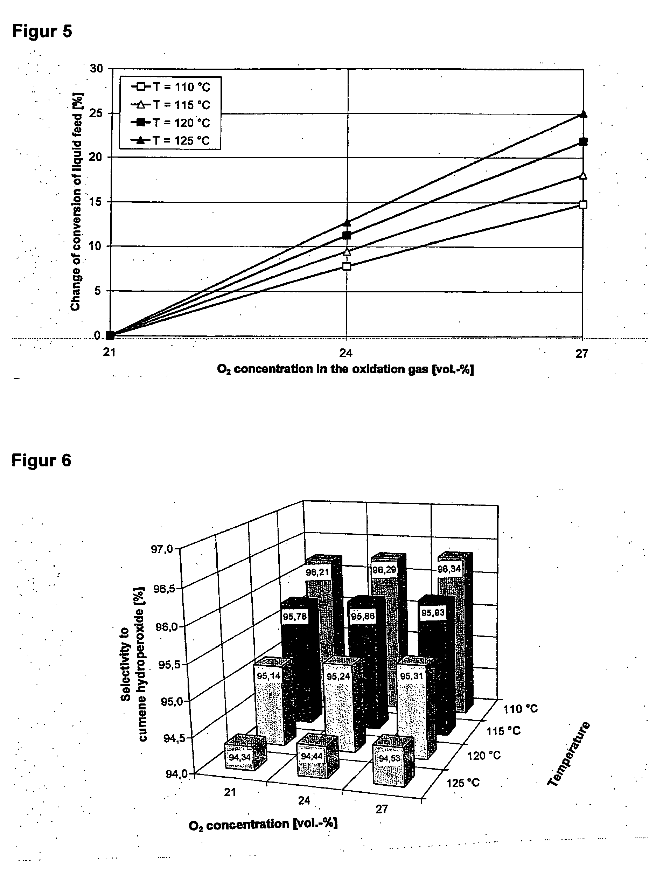

The kinetics and kinetic constants were determined based upon the results of the experiments and the work of Hattori (Hattori, K.; et al..: Kinetics of liquid phase oxidation of cumene in bubble column, Journal of Engineering of Japan Vol. 3 No. 1 1970 72 - 80). In this regard reference is made to Fig. 3b. By means of these macrokinetics and the determined rate constants, the experiments could be well replicated. The results of the experiments are shown in Fig. 4 to 6. In particular, in Fig. 4 and 5 the change in conversion of cumene depending on the oxygen content is shown. In Fig. 6 it is shown that at a constant temperature level the selectivity with respect to cumene hydroperoxide proves to be independent of the oxygen content in the oxidation air.

Table 3 shows the effects achievable through oxygen enrichment of the oxidation air to 24% or 27% by volume. The temperature in the reactor varied from 119 °C in the sump to 126 °C in the head of the bubble column.

**Table 3: Calculation results for oxygen content of 24% and 27% by volume while maintaining the oxygen concentration of 4.2% by volume in the effluent gas**

| | **Difference compared to air-only (i.e. 21% O₂) oxidation** | |
|---|---|---|
| | **24% by volume O₂** | **27% by volume O₂** |
| **Oxidation gas** | | |
| Volume flow [Nm³/h] | -11,0 % | -19,6 % |

| **Consumption / Production** | | |
|---|---|---|
| Converted cumene [kg/h] | + 4,6 % | + 8,6 % |
| Production of cumene hydroperoxide [kg/h] | + 4,7 % | + 8,7 % |
| Production of acetophenone [kg/h] | +2,6% | + 5,3 % |
| Production of dimethyl phenyl carbinol [kg/h] | + 3,1 % | + 5,7 % |

| **Effluent gas** | | |
|---|---|---|
| Volume flow [Nm³/h] | -15,2 % | -25,8 % |

The overall investigations and calculations for various technical reactors show that oxygen enrichment of the oxidation air of up to 27% by volume can have considerable economic effects. These include especially:
- Elevation of cumene hydroperoxide production by more than 8% at 4.2% by volume oxygen in the effluent gas.
- Reduction of the gas input flow by more than 19%
- Reduction of the effluent gas flow by more than 25%.

Financial analyses for one case involving a technical bubble column reactor have shown that the breakeven period is ≤ 1 year.

### Toluene oxidation

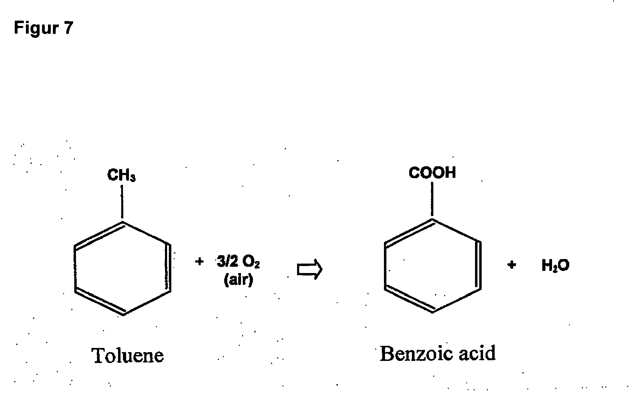

Air oxidation of toluene in the liquid phase is the most important process for manufacturing benzoic acid and often the by-product benzaldehyde is appreciated as a valuable bonus. Thereby applied temperature range is 120 to 180 °C at pressures of up to 10 bar and with a dissolved catalyst (such as cobalt naphthenate) (Ullmann's Encyclopedia of Industrial Chemistry:"Benzoic acid and derivatives"; 5^{th} Ed. 1985, Vol. A3, VCH-Verlag, p. 556 ff.). The main reaction is shown in Fig. 7.

Benzoic acid finds many direct applications, e.g. as preservative. But it also serves as a base chemical and as such being used for production of benzoate plasticizers, alkyd resins, n-butyl benzoate, sodium and potassium benzoate, benzoyl chloride. In some cases it acts as an intermediate in production of phenol and caprolactam.

The present experiments were performed in the experimental system described in Fig. 2 in order to investigate the influence of enhanced oxygen partial pressure on oxidation.

The most important experiment parameters were:

| | | |
|---|---|---|
| • | Temperature: | 115 -160 °C |
| • | Pressure: | 3 - 9 bar |
| • | Oxygen concentration in the oxidation air: | 21 to 27% by volume |
| • | Catalyst: | Co(II) naphthenate |

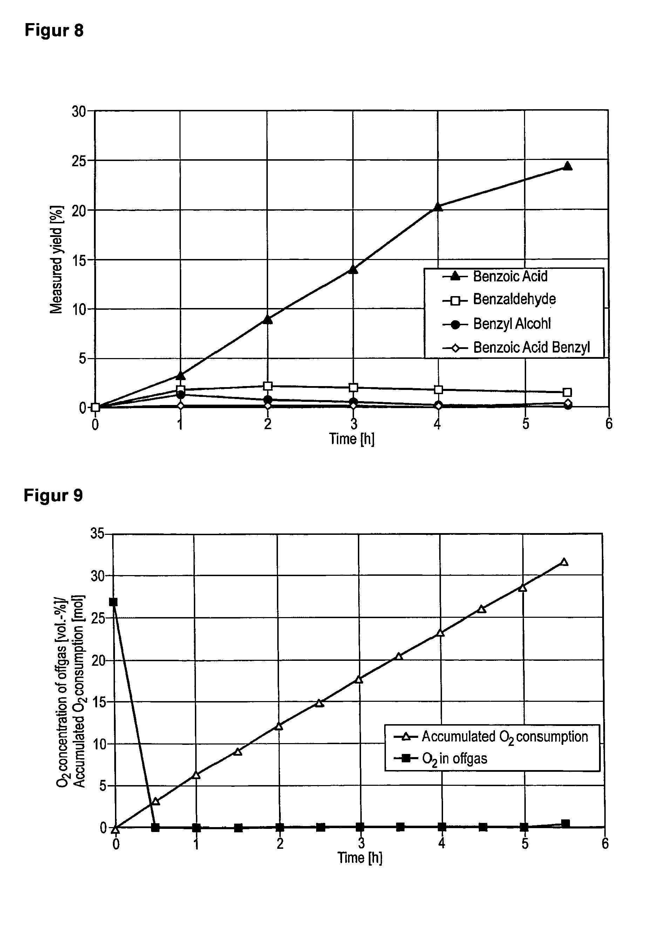

Figures 8 and 9 show some of the experimental results with an oxygen concentration of up to 27% by volume in the enriched oxidation air. At p/T- conditions of 9 barg/ 160°C already an enrichment level of 24% enhances conversion of toluene to benzoic acid significantly by more than 20% .

Figure 8 shows that two by-products run through a weak maximum and then even fall again somewhat. Overall in toluene oxidation, even when the concentration of oxygen in the enriched oxidation air is more elevated, the proportion of by-products is small.

Figure 9 shows that the level of oxygen in the effluent gas falls very quickly to a low concentration. As a result, the level is far away from the limiting concentration of oxygen. Yantowski et al. (International Chemical Engineering 7, 1967, p. 144) specify this limiting concentration as 6% by volume for a temperature range of 20 to 200 °C and a pressure range of 2 to 20 atm.

The experimental results can be summarized as follows:
1. By increasing the oxygen concentration in the oxidation air from 21% to 24% and 27% by volume with a temperature range of 140 to 160 °C and pressure of 9 bar, higher reaction speeds and conversion to benzoic acid could be achieved.
2. The selectivity to benzoic acid remains virtually constant as the concentration of oxygen increases.
3. The oxygen concentration of the effluent gas quickly sinks to a very small value.

Stirred tank reactors are often applied for toluene oxidation in the industrial scale. Accordingly the effects of oxygen enrichment were examined additionally for a technical stirred tank reactor. The calculation of the technical stirred tank reactor showed that at 160 °C the yield of benzoic acid increases by 30% when the oxygen concentration in the incoming air is increased from 21% to 27% by volume. In the effluent gas the limiting oxygen concentration of 6% by volume is not reached by this degree of oxygen enrichment.

### p-xylene oxidation for production of DMT

Purified terephthalic acid (PTA) and dimethyl terephthalate (DMT) are important raw materials for the production of polyesters. A portion is used as polyester resin for films, coatings, and adhesives as well as for the production of polyethylene terephthalate (PET), especially for beverage bottles. PET can be produced both from DMT and from PTA. In 2003, DMT production capacities were more than 4.9 million metric tons per year /1/. The realisable purity grade of DMT allows for high-grade quality in subsequent polyester production. The first commercial process for the oxidation of p-xylene was started in 1951. This process is also known as the Witten process (Dynamit Nobel process) and the basic concept has not changed since then.

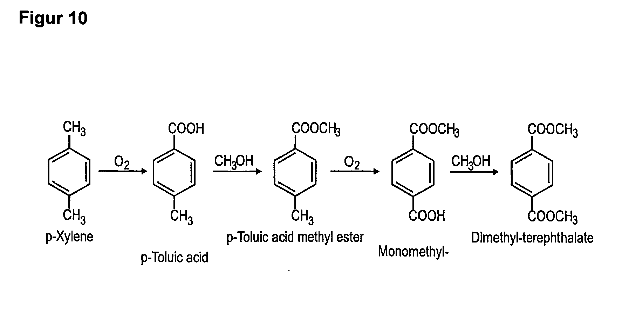

The core of the process is a liquid phase oxidation where a mixture of two compounds is oxidized at 140 to 170 °C, 4 to 8 bar, and in the presence of a dissolved Co/Mn salt as catalyst. Thereby only one of p-xylene's methyl groups can be oxidised and effective oxidation of the second methyl group is only possible after esterification of p-toluic acid with methanol. Due to this peculiarity yielded p-toluic acid methylester is recycled to the oxidation vessel and oxidised there together with the basic feed p-xylene. The reaction sequence shown in Fig. 10 gives an overview of the chemistry involved in the technical production of DMT.

As shown in Fig. 10 air oxidation of the liquid mixture of p-xylene (p-X) and p-toluic acid methylester (p-TE) primarily yields p-toluic acid (p-TA) and monomethyl terephthalate (MTT).

The experimental investigations of the influence of oxygen enrichment in this oxidation were performed in the stirred tank reactor system described in Fig. 2.

A mixture of p-xylene (p-X) and p-toluic acid methyl ester (p-TE) was used for oxidation as usual in the technical process for the production of DMT.

The reaction conditions were:

| | | |
|---|---|---|
| • | Temperature: | 150 to 170 °C |
| • | Pressure: | 6 and10 bar |
| • | Concentration of O₂ in the oxidation gas: | 21% to 27% by volume |
| • | Catalyst: | Co(II) acetate & Mn(II) acetate |

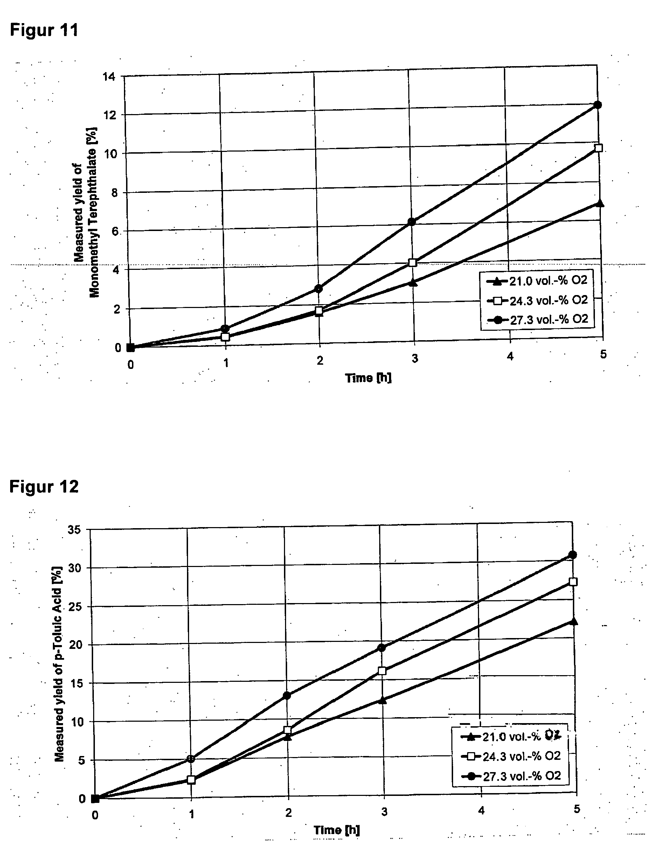

The results of the experiments (Fig. 11 and 12) can be summarized as follows: Increasing the concentration of oxygen in the air from 21 % to 24% and 27% by volume leads to higher reaction speeds and higher yields of the main oxidation products p-toluic acid (p-TA) and monomethyl terephthalate (MTT).

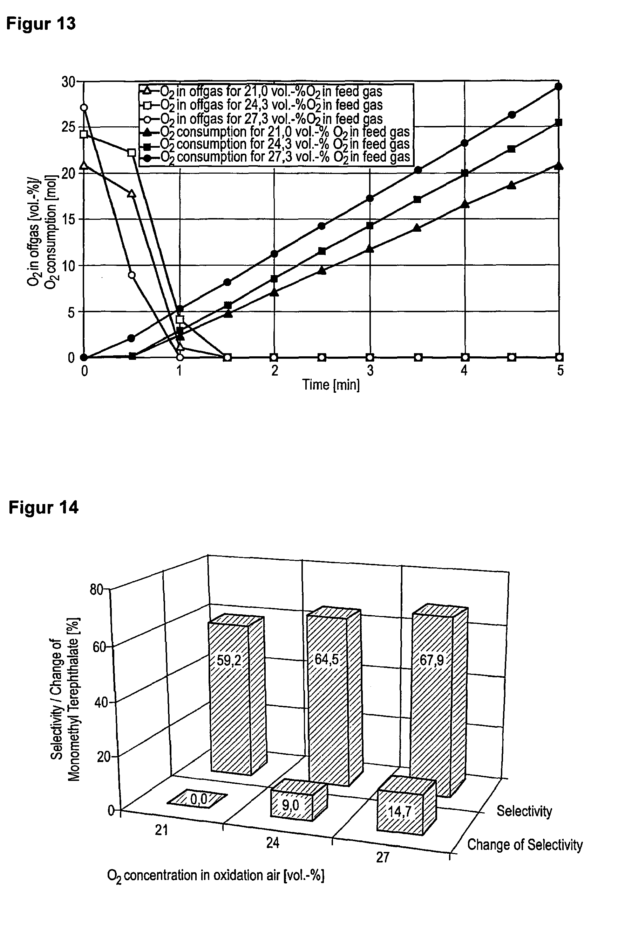

During all experiments, the concentration of oxygen in the effluent gas sank to values below the limiting concentration of oxygen (Figure 13). Values of between 0 and 1% by volume were measured. In other words, the limiting concentration of oxygen in the effluent gas does not represent an obstacle to oxygen enriched operation.

Figure 14 shows the calculated selectivities and selectivity changes as a function of the oxygen concentration of the oxidation air. The experiments with respect to oxygen enrichment in p-xylene oxidation have clearly shown that oxygen enrichment of up to 27% by volume increases space/time yield and can allow for significantly increased production of DMT. This possibility of process intensification is not only based on enhanced conversion but also due to improved selectivity.

### p-xylene oxidation for production of PTA

Global demand of purified terephthalic acid (PTA) in 2003 was more than 30 million metric tons; in 2005 demand was approximately 41 million metric tons. European capacities alone were 3.4 million metric tons in 2009. High growth rates are expected to continue mainly in Asia, especially in China.

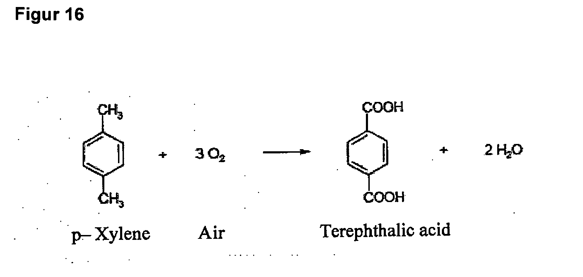

Applied production technologies - e.g. the Amoco process - trace back to the groundbreaking development of Scientific Design in the 1960ies. Here a special mixture of the liquid feed based on p-xylene and acetic acid as a solvent including highly corrosive bromine compounds (as initiator) in addition to dissolved cobalt and manganese salts is used. Due to this composition the air oxidation of p-xylene doesn't stop after yielding p-toluic acid but - other than in the Witten Process for DMT production - proceeds further to terephthalic acid according to the reaction in Fig. 16 an air oxidation which is highly exothermic.

Oxygen enrichment of the process air is used on a large scale in the production of PTA. Applied enrichment levels are in the range of 23% to 26% by volume. Here additional use of oxygen can achieve capacity increases of 12% to 30% and significantly reduce the effluent gas and thereby reduce the off-gas treatment.

## Claims

1. Method for the oxidation of alkyl aromatic hydrocarbons wherein the alkyl aromatic hydrocarbons are reacted with an oxidation gas, **characterized in that** the oxygen content in the oxidation gas is 21.5 to 30 % by volume.

2. The method of claim 1 **characterized in that** the alkyl aromatic hydrocarbon is cumene, toluene or p-xylene.

3. The method of claim 1 or 2 **characterized in that** the oxygen content in the oxidation gas is 24 to 27 % by volume.

4. The method of claim 2 or 3 **characterized in that** the oxidation product of cumene is cumene hydroperoxide and the oxidation is carried out at a reaction temperature between 105 °C and 125°C.

5. The method of claim 2 or 3 **characterized in that** the oxidation product of toluene is benzoic acid and the oxidation is carried out at a reaction temperature between 115 and 160°C.

6. The method of any of claims 1 to 5 **characterized in that** the oxidation gas is oxygen-enriched air or technical oxygen.

7. The method of any of claims 1 to 6 **characterized in that** the oxidation is carried out in a tank reactor system.

8. The method of any of claims 1 to 7 **characterized in that** the temperature of the oxidation gas is lower than the initialization temperature of the oxidation reaction of the alkyl aromatic hydrocarbons.
